# EUROPÄISCHE PATENTSCHRIFT

(11) **EP 3 538 543 B1**
(45) Veröffentlichungstag und Bekanntmachung des Hinweises auf die Patenterteilung: **08.06.2022**
(21) Anmeldenummer: 17800784.5
(22) Anmeldetag: 08.11.2017
(51) Int. Cl.: C07K 14/16, A61K 38/00, A61K 38/16

(54) **PROTRANSDUZIN-C - EIN ENHANCER DES GENTRANSFERS**
PROTRANSDUZIN-S - AN ENHANCER OF GENE TRANSFER
PROTRANSDUZIN-S - UN ACTIVATEUR DU TRANSFERT DE GÈNE

(30) Priorität: 09.11.2016 EP 16197999; 10.11.2016 EP 16198256
(43) Veröffentlichungstag der Anmeldung: 18.09.2019
(73) Patentinhaber: Pharis Biotec GmbH, 30625 Hannover (DE)
(72) Erfinder: FORSSMANN, Wolf-Georg, 30625 Hannover (DE); RICHTER, Rudolf, 30625 Hannover (DE)
(74) Vertreter: dompatent von Kreisler Selting Werner - Partnerschaft von Patent- und Rechtsanwälten mbB
(86) Internationale Anmeldenummer: PCT/EP2017/078600
(87) Internationale Veröffentlichungsnummer: WO 2018/087146

(56) Entgegenhaltungen:
- EP-A1- 2 452 947
- WO-A1-2014/177635
- MARAL YOLAMANOVA ET AL: "Peptide nanofibrils boost retroviral gene transfer and provide a rapid means for concentrating viruses", NATURE NANOTECHNOLOGY, Bd. 8, Nr. 2, 1. Februar 2013 (2013-02-01) , Seiten 130-136, XP055086479, ISSN: 1748-3387, DOI: 10.1038/nnano.2012.248

## Beschreibung

Gegenstand der vorliegenden Anmeldung ist ein Polypeptid, ein N-terminal geschütztes Polypeptid, ein Arzneimittel enthaltend das Polypeptid, das Polypeptid zur Verwendung in der Gentherapie, ein Verfahren zur Verstärkung der Infektion einer Zelle mit einem gentechnischen Viruskonstrukt, eine Verwendung des Polypeptids zur Amplifizierung zur Transfektion bzw. Transduktion. Alle Verweise auf Verfahren zur chirurgischen oder therapeutischen Behandlung des menschlichen oder tierischen Körpers, sind als Referenzen auf die Mittel, die pharmazeutischen Zusammensetzungen und Medikamente der vorliegenden Erfindung zur Verwendung in einem therapeutischen Verfahren zu interpretieren.

Die Bedeutung der Gentechnologie hat in den letzten Jahren durch enorme Fortschritte in den angewandten Methoden zugenommen, da nicht nur die Herstellung von Protein-Peptid-Wirkstoffen sondern auch die Transfektion von Zellen mit stabilen Genen als Labortool und zuletzt der Einschleusung von Genen in Zellen als Ersatz für Gendefekte von grosser Tragweite für die Therapie von zahlreichen Erkrankungen absehbar ist.

Die Einschleusung von Genmaterial zur Veränderung spezifischer Zellfunktionen ist seit der Klonierung der ersten humanen Gene und der rekombinanten Produktion ein unverzichtbares Werkzeug der biologisch-medizinischen Grundlagen- und Anwendungsforschung geworden. Dabei gibt sich ein ständiger Fortschritt der Methoden der Geneinschleusung, der zu einer Optimierung des Gentransfers führt. Erfahrungen, die über eine langjährige, zunächst sehr langsame Geschichte gesammelt wurden.

Schon vor der Aufklärung der Funktion von Desoxyribonukleinsäure (DNS) 1953 durch F. Crick und J. Watson, war es Ende der 1920' er Jahre F. Griffith in Experimenten gelungen, apathogene Pneumokokken-Stämme in Pathogene zu transformieren. Diese Transformation war einem glücklichen Umstand zu verdanken, da die Pneumokokken über eine seltene natürliche Kompetenz der DNS Aufnahme verfügten. Ein gezieltes Einschleusen der DNS in Prokaryonten gelang u.a. J. Lederberg, M. Delbrück und S. Luria mithilfe von Phagen, die so genannte Transduktion. Mit der Etablierung der Zellkultur, dem Kultivieren von Eukaryonten Zellen unter *in vitro* Bedingungen, wurden eine Reihe von physikalischen und chemischen Verfahren zur Transfektion entwickelt. Zu den häufiger genutzten, aber apparativ aufwendigeren, physikalischen Methoden gehören die Elektroporation und die Mikroinjektion, die mit den von der Anwendung einfacheren chemischen Verfahren konkurierten, wie die in den 80' er Jahren und auch noch heute übliche Calcium-Phosphat-Präzipitation Methode oder die in den frühen 90' er Jahren weit verbreiteten Methoden, die auf kationische Lipide bzw. kationische Polymere basierten. Der Einsatz dieser Verfahren war aber immer von den Zellen bzw. der DNS abhängig. Auch lag die in die Zellen eingeschleuste DNS im Allgemeinen extrachromosomal vor (transiente Transfektion) und wurde so nicht an die Tochterzellen weitergegeben. Von Phagen (z.B. Lambda Phage) wusste man aber, dass diese auch ihre DNS ins Wirtsgenom integrieren können (Prophage, lysogener Infektionsweg). Von hier aus war es nur noch ein kleiner Schritt zur (1981/1982) "Etablierung von Retroviren als Genvektoren" (durch Doehmer et al. und Tabin et al.). Viren sind Spezies- und Organ-/Gewebe- spezifisch, weshalb nicht alle Viren alle (Eukaryonten) Zellen infizieren. Veränderungen der Virus Hülle (Austausch von Glykoproteinen, sogenannte Pseudo-typed Viren) sowie Zusätze von meist kationischen Peptiden soll die Transduktionseffizienz steigern.

Erste Verstärker der Aufnahme von Viruspartikeln sind bei der Untersuchung von HIV aufgefallen. Bei Analysen der *in vitro* Infektion mittels eines spezifischen Zelltests wurde die Hemmung der Fusion von HIV durch Blutfiltratpeptide beobachtet. (Münch et al, VIRIP).

Es wurde gezeigt, dass diese überraschenderweise natürlich vorkommenden Fragmente von Proteinen im menschlichen Sperma faserartige Strukturen als enhancer - *"Semen derived Enhancer of Virus Infection"* (SEVI) ausbilden, die als Amyloidfibrillen gekennzeichnet sind. Diese Nanofibrillen verstärken das Andocken von Viren an ihre Zielzellen und erhöhen die Rate der Virusinfektion um mehrere log-Stufen.

Dies wurde genutzt, um einen retroviralen Gentransfer für die Grundlagenforschung und mögliche zukünftige therapeutische Anwendungen zu verbessern. So konnte gezeigt werden, dass lentivirale und gamma-retrovirale Vektoren, die für die Gentherapie verwendet werden, in der Anwesenheit des SEVI - Proteins eine vielfach höhere Gen-Transferrate bei unterschiedlichen Zelltypen aufweisen, wie z.B. humanen T-Zellen, Zervixkarzinomzellen, Leukämiezellen, hämatopoietischen Stammzellen und embryonalen Stammzellen (Wurm et al., J Gene Med. 2010, 12, 137-46; Wurm et al., Biol Chem. 2011, 392, 887-95).

Untersuchungen zur Entwicklung weiterer Enhancer wie z.B. dem SEVI und Seminogelin führten zu der Annahme, dass Peptide aus viralen Hüllproteinen auch als Enhancer der Transfektion geeignet sein könnten, was überraschenderweise von unerwartet gutem Erfolg war (Maral Yolamanova et al., Nature Nanotechnology, Bd. 8, Nr. 2, S. 130-136). So konnte z.B. gezeigt werden, dass HI-Viren, welche mit verschiedenen Konzentrationen (1-100 µg/ml) Protransduzin-A (Synonym: EF-C) vorinkubiert wurden, eine um mehrere log-Stufen stärkere Infektionsrate bei Reporterzellen aufweisen. Als Wirkmechanismus wurde angenommen, dass EF-C fibrilläre Strukturen ausbildet, die in der Lage sind, Viren zu binden, zu konzentrieren und entprechend den Zelleintritt der Viren zu vervielfachen. EF-C verstärkt neben der Infektion von Viruspartikeln mit hoher Effizienz die Transduktion von lentiviralen und retroviralen Partikeln in verschiedensten humanen Zelltypen (T-Zellen, Gliazellen, Fibroblasten, hämatopoietische Stammzellen), welche in der Gentherapie Anwendung finden (Maral Yolamanova et al., Nature Nanotechnology, Bd. 8, Nr. 2, S. 130-136). Protransduzin-A ist auch Gegenstand der EP 2 452 947 A1.

Aufgrund der oben dargestellten zunehmenden Bedeutung der Gentechnologie sind effektivere Verstärker des Gentransfers wünschenswert. Das der Erfindung zu Grunde liegende Problem ist die Bereitstellung eines verbesserten Verstärkers des Gentransfers.

Überraschenderweise wurde gefunden, dass Polypeptide gemäß Anspruch 1 das der Erfindung zu Grunde liegende Problem lösen. Bevorzugte Ausgestaltungen des Polypeptids sind in den Ansprüchen 2 und 3 beschrieben. Weitere Ausführungsformen sind in den Ansprüchen 4 bis 9 beansprucht.

Demnach betrifft die vorliegende Anmeldung ein Polypeptid zur Verstärkung der Transduktionseffizienz mit einer Sequenzidentität von mindestens 90 %, insbesondere 95 % zu der Sequenz wobei
die beiden Reste Z¹ das N-terminale Ende des Polypeptids oder die Aminosäuren Leu oder Ser oder die folgenden Peptide
Ser-Asn, Ser-Asn-Asn, Ser-Asn-Asn-Ile, Ser-Asn-Asn-Ile-Thr, Thr-Leu, Ile-Thr-Leu, Asn-Ile-Thr-Leu, Asn-Asn-Ile-Thr-Leu, oder Ser-Asn-Asn-Ile-Thr-Leu sind,
die beiden Reste Z² das C-terminale Ende des Polypeptids oder die Aminosäuren Gly oder Glu oder die folgenden Peptide
Glu-Val, Glu-Val-Gly, Glu-Val-Gly-Lys, Glu-Val-Gly-Lys-Ala, Glu-Val-Gly-Lys-Ala-Met, Glu-Val-Gly-Lys-Ala-Met-Tyr, Glu-Val-Gly-Lys-Ala-Met-Tyr-Ala, Glu-Val-Gly-Lys-Ala-Met-Tyr-Ala-Pro, Glu-Val-Gly-Lys-Ala-Met-Tyr-Ala-Pro-Pro, Glu-Val-Gly-Lys-Ala-Met-Tyr-Ala-Pro-Pro-Ile, Glu-Val-Gly-Lys-Ala-Met-Tyr-Ala-Pro-Pro-Ile-Glu, Glu-Val-Gly-Lys-Ala-Met-Tyr-Ala-Pro-Pro-Ile-Glu-Gly, Glu-Gly, Ile-Glu-Gly, Pro-Ile-Glu-Gly, Pro-Pro-Ile-Glu-Gly, Ala-Pro-Pro-Ile-Glu-Gly, Tyr-Ala-Pro-Pro-Ile-Glu-Gly, Met-Tyr-Ala-Pro-Pro-Ile-Glu-Gly, Ala-Met-Tyr-Ala-Pro-Pro-Ile-Glu-Gly, Lys-Ala-Met-Tyr-Ala-Pro-Pro-Ile-Glu-Gly, Gly-Lys-Ala-Met-Tyr-Ala-Pro-Pro-Ile-Glu-Gly, Val-Gly-Lys-Ala-Met-Tyr-Ala-Pro-Pro-Ile-Glu-Gly, oder Glu-Val-Gly-Lys-Ala-Met-Tyr-Ala-Pro-Pro-Ile-Glu-Gly sind.

Unter dem Begriff Sequenzidentität wird hier die Ähnlichkeit zwischen Proteinen (Aminosäuresequenzhomologie) aufgrund identischer Bausteinsequenzen in mehr oder weniger ausgedehnten Teilbereichen des Proteins verstanden. Die Sequenzidentität wird in Prozent identischer Positionen beim Vergleich zweier Peptidketten angegeben, wobei 100% Sequenzidentität völlige Identität der verglichenen Kettenmoleküle bedeutet. Bei Proteinen entsprechen 5% (100/20) identische Positionen der Zufallserwartung und entfallen damit zur Ableitung verwandtschaftlicher Beziehungen.

Die durch das erfindungsgemäße Polypeptid erzielbare Verbesserung geht mit einer erhöhten Effizienz der Gentransduktion in Zellen einher, die zu therapeutischen Zwecken eingesetzt werden können. Eine effizientere Gentransduktion in Zellen zur therapeutischen Anwendung kann z.B. die Menge an viralen Partikeln, die zur Gentransduktion verwendet werden, reduzieren. Weiterhin kann die Anzahl der Infektionzyklen, die für eine effiziente Transduktion notwendig sind, reduziert werden. Durch das erfindungsgemäße Polypeptid als Transduktionsverstärker kann die Dauer der in vitro Kultivierung zur Vermehrung der genmodifizierten Zellen, die Menge der vom Patienten zu entnehmenden Zellen (z.B. durch Leukapherese) reduziert und ggf. eine effiziente und nicht toxische *in vivo* Gentransduktion - durch Reduktion der Virusbelastung in vivo - ermöglicht werden. Weiterhin reduziert die schnelle Handhabung eines effizienten Transduktionsverstärkers die Belastung der zu transduzierenden Zellen.

Das erfindungsgemäße Polypeptid kann aus homologen oder heterologen Monomeren gebildet werden. Insbesondere ist das erfindungsgemäße Polypeptid ein aus zwei gleichen Monomeren über eine Disulfidbrücke verbundenes Dimere.

Insbesondere ist auch das Polypeptid mit einer Sequenzidentität von mindestens 90 % zu der Sequenz

### Gegenstand der Erfindung.

Erfindungsgemäß werden unter der Bezeichnung erfindungsgemäßes Polypeptid auch solche verwandten Polypeptide verstanden, die durch Variation der Aminosäuren in der Polypeptidkette des erfindungsgemäßen Polypeptids entstehen, aber noch eine vergleichbare und hinreichende Wirksamkeit aufweisen, die sich beispielsweise in folgendem Bioassay bestimmen lässt.

Die Transduktionseffizienz kann unter Verwendung von 293T Zellen als Targetzellen und lentiviralen und retroviralen Vektoren, die Green Fluoreszenz Protein (GFP) kodieren, getestet werden. Protransduzin-C wird in einem Assay in einer Konzentrationsreihe, z. B. in einer Konzentration von 0,01 bis 1000 µg/ml dem Assay eingesetzt. Die Targetzellen werden insbesondere in einer Konzentration von 10³ bis 10⁷ Zellen/ml eingesetzt. Für 8 bis 16 Stunden wird der Ansatz inkubiert und anschließend werden die Zellen gewaschen. Die Transduktionseffizienz wird anschließend anhand der GFP positiven Zellen mittels Durchflusszytometrie.

Ein sequenzähnliches (homologes) Polypeptid ist insbesondere ein mit der Aminosäuresequenz des erfindungsgemäßen Polypeptids verwandtes Polypeptid, bei dem im genannten Umfang Austausche oder Deletionen von Aminosäuren in der Aminosäurenkette vorgenommen wurden. Insbesondere sind Austausche von Aminosäuren, die ähnliche Eigenschaften haben, beispielsweise ähnliche Polaritäten, möglich. So sind Austausche von Arginin und Lysin, Glutaminsäure und Asparaginsäure, Glutamin, Asparagin und Threonin, Glycin, Alanin und Prolin, Leucin, Isoleucin und Valin, Thyrosin, Phenylalanin und Tryptophan sowie Serin und Threonin weit verbreitet.

Unter Sequenzhomologie wird hier auch folgendes verstanden: das erfindungsgemäße Polypeptid besteht aus zwei sequenzähnlichen oder identischen Monomeren, die jeweils die Aminosäure Cystein enthalten, über die die beiden Monomere über eine Disulfidbrücke miteinander verknüpft sind. Insbesondere besetzt die Aminosäure Cystein die Position 2 des Monomers. Alternativ können zur Verbrückung der Monomere auch andere kovalente Bindungen zwischen zwei Aminosäuren verwendet werden.

In Position 1 der Sequenz findet sich bevorzugt die Aminosäure Glutamin. In Position 3 und 5 finden sich bevorzugt basische Aminosäuren, bevorzugt Lysin. In den Positionen 1, 4, 6, 7, 8, 9, 10, 11, und 12 finden sich zumeist neutrale Aminosäuren. N- und/oder C-terminal kann die Sequenz verlängert oder verkürzt sein. N-terminal kann die Sequenz des Monomers um C-terminale Teile oder die gesamte Aminosäuresequenz NH₂-Ser-Asn-Asn-Ile-Thr-Leu-COOH verlängert sein. C-terminal kann die Sequenz des Monomers um N-terminale Teile oder die gesamte Aminosäuresequenz NH₂-Glu-Val-Gly-Lys-Ala-Met-Tyr-Ala-Pro-Pro-Ile-Glu-Gly-COOH verlängert sein. Den Peptiddimeren ist gemein, dass sie in wässrigen Lösungen unlösliche Aggregate bilden. Die Monomere bestehen aus 4 bis 25 Aminosäuren, bevorzugt aber aus 10 bis 20 Aminosäuren.

Sequenzähnliche Polypeptide weisen eine Sequenzidentität von mindestens 90%, insbesondere 95% auf. Strukturell sind bei den sequenzähnlichen Molekülen die Dimerisierung und mindestens zwei basische Aminosäuren zu finden. Weiterhin ist den sequenzähnlichen Molekülen gemein, dass sie in wässrigen Lösungen unlösliche Aggregate bilden und die Transduktion von Targetzellen mit lentiviralen oder retroviralen Vektoren steigern.

In einer alternativen Ausführungsform der Erfindung ist mindestens ein N-terminales Ende der beiden das erfindungsgemäße Polypeptid bildenden Aminosäureketten mit einer chemischen Gruppe modifiziert, die ausgewählt ist aus der Gruppe bestehend aus einer oder zwei Alkylgruppen, wie Methyl-, Ethyl-, Propyl- oder Butylgruppen, eine Acylgruppe, wie eine Acetyl- oder Propionylgruppe. Alternativ kann die N-terminale Aminosäure durch eine Pyroglutaminsäure ausgetauscht sein, so dass die Pyroglutaminsäure 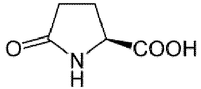 das N-terminale Ende bildet.

Gegenstand der Erfindung ist auch ein Hilfsstoff zur Präzipitation von Viren enthaltend mindestens ein erfindungsgemäßes Polypeptid. Durch den Einsatz des erfindungsgemäßen Polypeptids als Präzipitationshilfsstoff kann die Isolierung von Viren durch Zentrifugation erheblich erleichtert werden, weil die anzuwendenden Zentrifugationszeiten deutlich verkürzt werden können.

Gegenstand der Erfindung ist auch ein Arzneimittel enthaltend mindestens ein erfindungsgemäßes Polypeptid.

Gegenstand der Erfindung ist ebenfalls die Verwendung eines erfindungsgemäßen Polypeptides in der Gentherapie zur Behandlung von genetisch bedingten Erkrankungen. Als Gentherapie bezeichnet man in der Medizin das Einfügen von Genen in Zellen oder Gewebe eines Menschen, um Erbkrankheiten oder Gendefekte zu behandeln.

Ebenfalls ist Gegenstand der vorliegenden Erfindung ein Verfahren zur Verstärkung der Infektion einer Zelle mit einem Virus mit den Schritten:
- Bereitstellen des Polypeptids gemäß Anspruch 1 oder 2 gelöst in einem organischen Lösungsmittel,
- Zugabe des Polypeptids zu einer wässrigen Lösung, unter Bildung von unlösbaren Aggregaten des Polypeptids,
- Vermischen der Lösung aus dem vorhergehenden Schritt und
- Kultivieren von Zellen in Anwesenheit mindestens eines erfindungsgemäßen Polypeptids.

Gegenstand der Erindung ist auch die Verwendung mindestens eines erfindungsgemäßen Polypeptids zur Verstärkung der Infektion von Zellen mit einem Virus.

Eine Zusammensetzung enthaltend mindestens ein erfindungsgemäßes Polypeptid ist ebenfalls Gegenstand der vorliegenden Erfindung.

Das erfindungsgemäße Polypeptid kann beispielsweise mit der Methode nach Merrifield mit Fmoc geschützten Aminosäuren synthetisiert werden.

Dabei wird mit Fmoc-geschützten Derivaten gearbeitet, also mit (9-fluorenylmethoxycarbonyl) -geschützten Aminosäuren in einer schrittweisen Festphasensynthese nach dem Merrifield-Prinzip, insbesondere auf einem mit Fmoc-L-Glutamin vorbeladenem Wang-Harz (0,59 mmol/g, 100 - 200 mesh) als festem Träger am Synthesizer ABI-433.

Die Aktivierung der Fmoc-L-Aminosäuren, die typischerweise in 10-fachem molaren Überschuss eingesetzt wurden, werden mit [(2-(1H-Benzotriazol-1-yl)-1,1,3,3-tetramethyluronium-hexafluorophosphat] (HBTU, 100 mmol/l) unter Zusatz von 0.5 M 1-Hydroxy-benzotriazol (HOBt) und 2M Diisopropylethylamin (DIEA) in N-Methyl-2-pyrrolidinon (NMP) bei Raumtemperatur durchgeführt.

Die Acylierungsreaktionen dauern im Einzelnen 45 Minuten, die Abspaltung der Fmoc- Schutzgruppe mit 20 %igem Piperidin dauert 15 Minuten.

Folgende Aminosäurederivate sowie dazugehörige orthogonal-säurelabile Seitenkettenschutzgruppen werden zur Synthese eingesetzt:
Fmoc-L-Asn(Trt), Fmoc-L-Cys(Trt), L-pGlu, Fmoc-L-Gln(Trt), Fmoc-L-Ile, Fmoc-L-Lys(Boc), Fmoc-L-Met und Fmoc-L-Trp(Boc).

Nach Abspaltung des Harzträgers vom Peptidylharz mit 94 %iger Trifluoressigsäure (TFA); 3%igem Ethandithiol (EDT) und 3 %igem entmineralisiertem Wasser wird das Rohpeptid in kaltem tert. Butylmethylether ausgefällt, das Rohpeptid als Sediment abzentrifugiert, der Überstand verworfen.

Die anschließende chromatographische Aufreinigung des Rohpeptides erfolgt präperativ per Gradientenelution.

Der Unterschied von Protransduzin-A gemäß EP 2 452 947 A1 und Protransduzin-B gemäß WO 2014/177635 A1 zu Protransduzin-C ist der, dass Protransduzin-C erfindungsgemäß aus zwei Peptidmonomeren besteht, die über eine Disulfidbrücke kovalent zu einem Peptiddimer verknüpft sind.

Beispiel:
Transduktion von 293 T Zellen mit Protransduzin-C

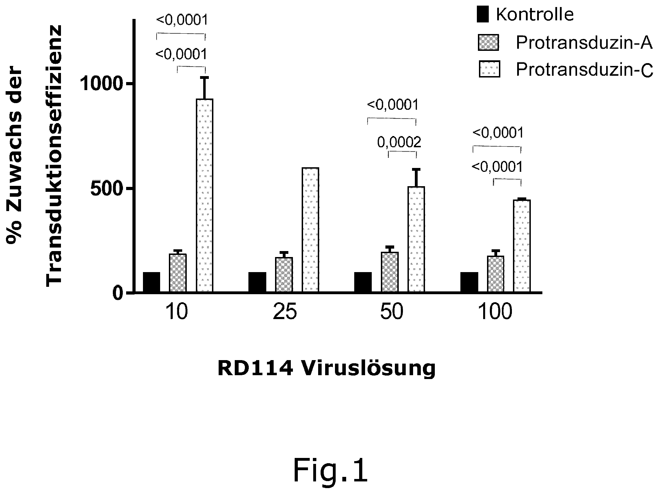

293T Zellen werden in 12 Well Kulturplatten ausgesäht und für 16 h kultiviert. Je Well werden 200.000 293T Zellen eingesetzt. Nach 16 Stunden werden ein Vial mit 1,0 mg Protransduzin-C und ein Vial mit 1,0 mg Protransduzin-A mit je 100 µl DMSO versetzt und Protransduzin-C und Protransduzin-A damit vollständig gelöst. Danach werden in jedes Vial jeweils 900 µl PBS zugefügen. Dabei entstehen innerhalb von 3 min. Fibrillen. Anschließend werden Protransduzin-C oder Protransduzin-A in einer Endkonzentration von 25µg/ml den Kulturwells hinzugegeben. Dann werden verschiedene Mengen an RD114 Virus Stock (10µl, 25 µl, 50 µl, 100µl) hinzugegeben und das fehlende Volumen mit DMEM auf 500µl aufgefüllt. Die verwendeten RD114 Viren kodieren das Green Fluoreszent Protein. Zur Steigerung der Transduktionsrate werden die Ansätze bei 600 g für 1 h bei 25°C zentrifugiert. Nach 6 h werden 500 µl Zellkulturmedium hinzugegeben. 24 h nach Transduktion wird das Zellkulturmedium gewechselt. 48 h nach Transduktion wird die Transduktionseffizienz in der Durchflusszytometrie durch Bestimmung der Expression des Green Fluorescence Protein in den 293T Zellen bestimmt. In den Experimenten zeigt sich eine signifikant höhere Potenz von Protransduzin-C zur Verstärkung der Transduktion im Vergleich zu Protransduzin-A und zur Kontrolle (Fig. 1).

Protransduzin-B weist eine ähnliche Transduktionseffizienz wie das Protransduzin-A auf, sodass die Transduktionseffizienz von Protransduzin-C im Vergleich zu Protransduzin-B ebenfalls signifikant erhöht ist, ähnlich zu den Resultaten gemäß Fig. 1.

## Patentansprüche

1. Polypeptid zur Verstärkung der Transduktionseffizienz mit einer Sequenzidentität von mindestens 90 % zu der Aminosäuresequenz
wobei die beiden Reste Z¹ das N-terminale Ende des Poypeptids oder die Aminosäuren Leu oder Ser oder die folgenden Peptide
Ser-Asn, Ser-Asn-Asn, Ser-Asn-Asn-Ile, Ser-Asn-Asn-Ile-Thr, Thr-Leu, Ile-Thr-Leu, Asn-Ile-Thr-Leu, Asn-Asn-Ile-Thr-Leu, oder Ser-Asn-Asn-Ile-Thr-Leu sind,
und die beiden Reste Z² das C-terminale Ende des Polypeptids oder die Aminosäuren Gly oder Glu oder die folgenden Peptide
Glu-Val, Glu-Val-Gly, Glu-Val-Gly-Lys, Glu-Val-Gly-Lys-Ala, Glu-Val-Gly-Lys-Ala-Met, Glu-Val-Gly-Lys-Ala-Met-Tyr, Glu-Val-Gly-Lys-Ala-Met-Tyr-Ala, Glu-Val-Gly-Lys-Ala-Met-Tyr-Ala-Pro, Glu-Val-Gly-Lys-Ala-Met-Tyr-Ala-Pro-Pro, Glu-Val-Gly-Lys-Ala-Met-Tyr-Ala-Pro-Pro-Ile, Glu-Val-Gly-Lys-Ala-Met-Tyr-Ala-Pro-Pro-Ile-Glu, Glu-Val-Gly-Lys-Ala-Met-Tyr-Ala-Pro-Pro-Ile-Glu-Gly, Glu-Gly, Ile-Glu-Gly, Pro-Ile-Glu-Gly, Pro-Pro-Ile-Glu-Gly, Ala-Pro-Pro-Ile-Glu-Gly, Tyr-Ala-Pro-Pro-Ile-Glu-Gly, Met-Tyr-Ala-Pro-Pro-Ile-Glu-Gly, Ala-Met-Tyr-Ala-Pro-Pro-Ile-Glu-Gly, Lys-Ala-Met-Tyr-Ala-Pro-Pro-Ile-Glu-Gly, Gly-Lys-Ala-Met-Tyr-Ala-Pro-Pro-Ile-Glu-Gly, Val-Gly-Lys-Ala-Met-Tyr-Ala-Pro-Pro-Ile-Glu-Gly, oder Glu-Val-Gly-Lys-Ala-Met-Tyr-Ala-Pro-Pro-Ile-Glu-Gly sind.

2. Polypeptid zur Verstärkung der Transduktionseffizienz nach Anspruch 1 mit einer Sequenzidentität von mindestens 90 % zu der Sequenz

3. Polypeptid zur Verstärkung der Transduktionseffizienz nach Anspruch 1 oder 2, wobei mindestens ein N-terminales Ende der beiden das Polypeptid bildenden Aminosäureketten mit einer chemischen Gruppe modifiziert ist, die ausgewählt ist aus der Gruppe bestehend aus einer oder zwei Alkylgruppen, wie Methyl-, Ethyl-, Propyl- oder Butylgruppen, eine Acylgruppe, wie eine Acetyl- oder Propionylgruppe oder die Aminosäure Pyroglutaminsäure das N-terminale Ende bildet.

4. Hilfsstoff zur Präzipitation von Viren enthaltend ein Polypeptid gemäß einem der Ansprüche 1 bis 3.

5. Arzneimittel enthaltend ein Polypeptid gemäß einem der Ansprüche 1 bis 3.

6. Polypeptid nach einem der Ansprüche 1 bis 3 zur Verwendung in der Gentherapie zur Behandlung von genetisch bedingten Erkrankungen.

7. Verfahren zur Verstärkung der Infektion einer Zelle mit einem Virus mit den Schritten:
- Bereitstellen des Polypeptids gemäß einem der Ansprüche 1 bis 3 gelöst in einem organischen Lösungsmittel,
- Zugabe des Polypeptids zu einer wässrigen Lösung, unter Bildung von unlösbaren Aggregaten des Polypeptids,
- Vermischen der Lösung aus dem vorhergehenden Schritt und
- Kultivieren von Zellen in Anwesenheit des Polypeptids gemäß einem der Ansprüche 1 bis 3.

8. In vitro Verwendung des Polypeptids gemäß einem der Ansprüche 1 bis 3 zur Verstärkung der Infektion einer Zelle mit einem Virus.

9. Zusammensetzung enthaltend ein Polypeptid gemäß mindestens einem der Ansprüche 1 bis 3.

## Claims

1. A polypeptide for enhancing transduction efficiency having a sequence identity of at least 90%, to the amino acid sequence wherein
both residues Z¹ are the N-terminal end of the polypeptide, or the amino acids Leu or Ser, or the following peptides
Ser-Asn, Ser-Asn-Asn, Ser-Asn-Asn-Ile, Ser-Asn-Asn-Ile-Thr, Thr-Leu, Ile-Thr-Leu, Asn-Ile-Thr-Leu, Asn-Asn-Ile-Thr-Leu, or Ser-Asn-Asn-Ile-Thr-Leu,
and both residues Z² are the C-terminal end of the polypeptide, or the amino acids Gly or Glu, or the following peptides
Glu-Val, Glu-Val-Gly, Glu-Val-Gly-Lys, Glu-Val-Gly-Lys-Ala, Glu-Val-Gly-Lys-Ala-Met, Glu-Val-Gly-Lys-Ala-Met-Tyr, Glu-Val-Gly-Lys-Ala-Met-Tyr-Ala, Glu-Val-Gly-Lys-Ala-Met-Tyr-Ala-Pro, Glu-Val-Gly-Lys-Ala-Met-Tyr-Ala-Pro-Pro, Glu-Val-Gly-Lys-Ala-Met-Tyr-Ala-Pro-Pro-Ile, Glu-Val-Gly-Lys-Ala-Met-Tyr-Ala-Pro-Pro-Ile-Glu, Glu-Val-Gly-Lys-Ala-Met-Tyr-Ala-Pro-Pro-Ile-Glu-Gly, Glu-Gly, Ile-Glu-Gly, Pro-Ile-Glu-Gly, Pro-Pro-Ile-Glu-Gly, Ala-Pro-Pro-Ile-Glu-Gly, Tyr-Ala-Pro-Pro-Ile-Glu-Gly, Met-Tyr-Ala-Pro-Pro-Ile-Glu-Gly, Ala-Met-Tyr-Ala-Pro-Pro-Ile-Glu-Gly, Lys-Ala-Met-Tyr-Ala-Pro-Pro-Ile-Glu-Gly, Gly-Lys-Ala-Met-Tyr-Ala-Pro-Pro-Ile-Glu-Gly, Val-Gly-Lys-Ala-Met-Tyr-Ala-Pro-Pro-Ile-Glu-Gly, or Glu-Val-Gly-Lys-Ala-Met-Tyr-Ala-Pro-Pro-Ile-Glu-Gly.

2. The polypeptide for enhancing transduction efficiency according to claim 1, with a sequence identity of at least 90% to the sequence

3. The polypeptide for enhancing transduction efficiency according to claim 1 or 2, wherein at least one N-terminal end of the two amino acid chains forming the polypeptide is modified with a chemical group selected from the group consisting of one or two alkyl groups, such as methyl, ethyl, propyl or butyl groups, an acyl group, such as an acetyl or propionyl group, or the amino acid pyroglutamic acid forms the N-terminal end.

4. An auxiliary agent for the precipitation of viruses, containing a polypeptide according to any of claims 1 to 3.

5. A medicament containing a polypeptide according to any of claims 1 to 3.

6. A polypeptide according to any of claims 1 to 3 for use in gene therapy for treating genetically caused diseases.

7. A process for enhancing the infection of a cell with a virus, comprising the steps of:
- providing the polypeptide according to any of claims 1 to 3 dissolved in an organic solvent;
- adding the polypeptide to an aqueous solution to form insoluble aggregates of the polypeptide;
- mixing the solution from the preceding step; and
- culturing cells in the presence of the polypeptide according to any of claims 1 to 3.

8. *In vitro* use of the polypeptide according to any of claims 1 to 3 for enhancing the infection of a cell with a virus.

9. A composition containing a polypeptide according to at least one of claims 1 to 3.

## Revendications

1. Polypeptide pour augmenter l'efficacité de transduction ayant une identité de séquence d'au moins 90 % avec la séquence d'acides aminés suivante : où
les deux résidus Z¹ sont l'extrémité N-terminale du polypeptide, ou les acides aminés Leu ou Ser, ou les peptides suivants :
Ser-Asn, Ser-Asn-Asn, Ser-Asn-Asn-Ile, Ser-Asn-Asn-Ile-Thr, Thr-Leu, Ile-Thr-Leu, Asn-Ile-Thr-Leu, Asn-Asn-Ile-Thr-Leu, ou Ser-Asn-Asn-Ile-Thr-Leu,
et les deux résidus Z² sont l'extrémité C-terminale du polypeptide, ou les acides aminés Gly ou Glu, ou les peptides suivants :
Glu-Val, Glu-Val-Gly, Glu-Val-Gly-Lys, Glu-Val-Gly-Lys-Ala, Glu-Val-Gly-Lys-Ala-Met, Glu-Val-Gly-Lys-Ala-Met-Tyr, Glu-Val-Gly-Lys-Ala-Met-Tyr-Ala, Glu-Val-Gly-Lys-Ala-Met-Tyr-Ala-Pro, Glu-Val-Gly-Lys-Ala-Met-Tyr-Ala-Pro-Pro, Glu-Val-Gly-Lys-Ala-Met-Tyr-Ala-Pro-Pro-Ile, Glu-Val-Gly-Lys-Ala-Met-Tyr-Ala-Pro-Pro-Ile-Glu, Glu-Val-Gly-Lys-Ala-Met-Tyr-Ala-Pro-Pro-Ile-Glu-Gly, Glu-Gly, Ile-Glu-Gly, Pro-Ile-Glu-Gly, Pro-Pro-Ile-Glu-Gly, Ala-Pro-Pro-Ile-Glu-Gly, Tyr-Ala-Pro-Pro-Ile-Glu-Gly, Met-Tyr-Ala-Pro-Pro-Ile-Glu-Gly, Ala-Met-Tyr-Ala-Pro-Pro-Ile-Glu-Gly, Lys-Ala-Met-Tyr-Ala-Pro-Pro-Ile-Glu-Gly, Gly-Lys-Ala-Met-Tyr-Ala-Pro-Pro-Ile-Glu-Gly, Val-Gly-Lys-Ala-Met-Tyr-Ala-Pro-Pro-Ile-Glu-Gly, ou Glu-Val-Gly-Lys-Ala-Met-Tyr-Ala-Pro-Pro-Ile-Glu-Gly.

2. Polypeptide pour augmenter l'efficacité de transduction selon la revendication 1, ayant une identité de séquence d'au moins 90 % avec la séquence suivante :

3. Polypeptide pour augmenter l'efficacité de transduction selon la revendication 1 ou 2, dans lequel au moins une extrémité N-terminale des deux chaînes d'acides aminés formant le polypeptide est modifiée avec un groupe chimique choisi dans le groupe consistant en un ou deux groupes alkyle, tels que des groupes méthyle, éthyle, propyle ou butyle, un groupe acyle, tel qu'un groupe acétyle ou propionyle, ou l'acide aminé acide pyroglutamique forme l'extrémité N-terminale.

4. Adjuvant pour la précipitation de vires contenant un polypeptide selon l'une quelconque des revendications 1 à 3.

5. Médicament contenant un polypeptide selon l'une quelconque des revendications 1 à 3.

6. Polypeptide selon l'une quelconque des revendications 1 à 3 à utiliser dans la thérapie génique pour traiter des maladies d'origine génétique.

7. Procédé pour augmenter l'infection d'une cellule avec un virus, comprenant les étapes consistant à
- procurer le polypeptide selon l'une quelconque des revendications 1 à 3 dissout dans un solvant organique,
- ajouter le polypeptide à une solution aqueuse pour former des agrégats insolubles du polypeptide,
- mélanger la solution provenant de l'étape ci-dessus, et
- cultiver des cellules en présence du polypeptide selon l'une quelconque des revendications 1 à 3.

8. Utilisation *in-vitro* du polypeptide selon l'une quelconque des revendications 1 à 3 pour augmenter l'infection d'une cellule avec un virus.

9. Composition contenant un polypeptide selon au moins l'une des revendications 1 à 3.


### SEQUENCE LISTING

<110> Pharis Biotec GmbH
<120> Protransduzin-C - ein Enhancer des Gentransfers
<130> 172247WO
<150> EP16197999
   <151> 2016-11-09
<160> 25
<170> PatentIn version 3.5
<210> 1
   <211> 12
   <212> PRT
   <213> human
<400> 1
   Gln Cys Lys Ile Lys Gln Ile Ile Asn Met Trp Gln
   1               5                   10
<210> 2
   <211> 4
   <212> PRT
   <213> human
<400> 2
   Ser Asn Asn Ile
   1
<210> 3
   <211> 5
   <212> PRT
   <213> human
<400> 3
   Ser Asn Asn Ile Thr
   1               5
<210> 4
   <211> 5
   <212> PRT
   <213> human
<400> 4
   Asn Asn Ile Thr Leu
   1               5
<210> 5
   <211> 6
   <212> PRT
   <213> human
<400> 5
   Ser Asn Asn Ile Thr Leu
   1               5
<210> 6
   <211> 4
   <212> PRT
   <213> human
<400> 6
   Glu Val Gly Lys
   1
<210> 7
   <211> 5
   <212> PRT
   <213> human
<400> 7
   Glu Val Gly Lys Ala
   1               5
<210> 8
   <211> 6
   <212> PRT
   <213> human
<400> 8
   Glu Val Gly Lys Ala Met
   1               5
<210> 9
   <211> 7
   <212> PRT
   <213> human
<400> 9
   Glu Val Gly Lys Ala Met Tyr
   1               5
<210> 10
   <211> 8
   <212> PRT
   <213> human
<400> 10
   Glu Val Gly Lys Ala Met Tyr Ala
   1               5
<210> 11
   <211> 9
   <212> PRT
   <213> human
<400> 11
   Glu Val Gly Lys Ala Met Tyr Ala Pro
   1 5
<210> 12
   <211> 10
   <212> PRT
   <213> human
<400> 12
   Glu Val Gly Lys Ala Met Tyr Ala Pro Pro
   1 5 10
<210> 13
   <211> 11
   <212> PRT
   <213> human
<400> 13
   Glu Val Gly Lys Ala Met Tyr Ala Pro Pro Ile
   1 5 10
<210> 14
   <211> 12
   <212> PRT
   <213> human
<400> 14
   Glu Val Gly Lys Ala Met Tyr Ala Pro Pro Ile Glu
   1 5 10
<210> 15
   <211> 13
   <212> PRT
   <213> human
<400> 15
   Glu Val Gly Lys Ala Met Tyr Ala Pro Pro Ile Glu Gly
   1 5 10
<210> 16
   <211> 4
   <212> PRT
   <213> human
<400> 16
   Pro Ile Glu Gly
   1
<210> 17
   <211> 5
   <212> PRT
   <213> human
<400> 17
   Pro Pro Ile Glu Gly
   1 5
<210> 18
   <211> 6
   <212> PRT
   <213> human
<400> 18
   Ala Pro Pro Ile Glu Gly
   1 5
<210> 19
   <211> 7
   <212> PRT
   <213> human
<400> 19
   Tyr Ala Pro Pro Ile Glu Gly
   1 5
<210> 20
   <211> 8
   <212> PRT
   <213> human
<400> 20
   Met Tyr Ala Pro Pro Ile Glu Gly
   1 5
<210> 21
   <211> 9
   <212> PRT
   <213> human
<400> 21
   Ala Met Tyr Ala Pro Pro Ile Glu Gly
   1 5
<210> 22
   <211> 10
   <212> PRT
   <213> human
<400> 22
   Lys Ala Met Tyr Ala Pro Pro Ile Glu Gly
   1 5 10
<210> 23
   <211> 11
   <212> PRT
   <213> human
<400> 23
   Gly Lys Ala Met Tyr Ala Pro Pro Ile Glu Gly
   1               5                   10
<210> 24
   <211> 12
   <212> PRT
   <213> human
<400> 24
   Val Gly Lys Ala Met Tyr Ala Pro Pro Ile Glu Gly
   1               5                   10
<210> 25
   <211> 13
   <212> PRT
   <213> human
<400> 25
   Glu Val Gly Lys Ala Met Tyr Ala Pro Pro Ile Glu Gly
   1               5                   10